Europäisches Patentamt

European Patent Office

Office européen des brevets

(19)

(11) Veröffentlichungsnummer: **0 058 829**
**B1**

(12) # EUROPÄISCHE PATENTSCHRIFT

(45) Veröffentlichungstag der Patentschrift:
17.04.85

(21) Anmeldenummer: **82100476.9**

(22) Anmeldetag: **23.01.82**

(51) Int. Cl.⁴: **A 61 F  5/44,** A 61 M  1/00

(54) **Tropfkammer in einem Ableitungssystem für Körpersekret, insbesondere Urin.**

(30) Priorität: **21.02.81  DE 3106488**

(43) Veröffentlichungstag der Anmeldung:
**01.09.82 Patentblatt 82/35**

(45) Bekanntmachung des Hinweises auf die Patenterteilung:
**17.04.85 Patentblatt 85/16**

(84) Benannte Vertragsstaaten:
**AT BE CH DE FR GB IT LI LU NL SE**

(56) Entgegenhaltungen:
**DE - A - 2 227 706**
**DE - A - 2 435 288**
**DE - A - 2 545 295**
**US - A - 4 198 971**

(73) Patentinhaber: **INTERMEDICAT GMBH,**
**Gerliswilstrasse 43, CH-6020 Emmenbrücke (CH)**

(72) Erfinder: **Haacke, Claus, Dr. Dipl.-Chem., Altstadt 6,**
**D-3508 Melsungen (DE)**

(74) Vertreter: **von Kreisler, Alek, Dipl.-Chem. et al,**
**Deichmannhaus am Hauptbahnhof, D-5000 Köln 1 (DE)**

EP 0 058 829 B1

## Beschreibung

Die Erfindung bezieht sich auf eine Tropfkammer in einem Ableitungssystem für Körpersekret, insbesondere Urin, mit einem im wesentlichen starren Gehäuse, dessen unterer Auslaß über ein Ventil an einen Sammel- oder Meßbehälter angeschlossen ist und dessen oberer Einlaß ein in das Tropfkammergehäuse hineinragendes Tropfrohr aufweist, das mit einer Körpersekretableitung verbunden ist.

Eine Tropfkammer hat in einem Ableitungssystem für Körpersekret, insbesondere Urin, die Aufgabe, den Körpersekretfluß zwischen dem Tropfrohr und dem Sammel- oder Meßbehälter zu unterbrechen und zur Reduktion von kanalikulär erzeugten, das heißt retrograd aufgestiegenen, Infektionen und ihren Folgen eine Keimbarriere zu bilden. Dieser Zweck wird nur dann erfüllt, wenn die Tropfkammer im Bereich der Spitze des Tropfrohres trocken bleibt und nicht von rückströmendem Urin benetzt wird. Solange die Tropfkammer und der an sie angeschlossene Sammel- oder Meßbehälter z. B. ein Sammelbeutel aus Folienmaterial, senkrecht hängen und nicht gekippt werden, besteht keine wesentliche Gefahr der Kontamination der Spitze des Tropfrohres, weil es von einem Ringraum umgeben ist, der das Tropfrohr im Abstand zu der Wand des Tropfkammergehäuses hält (DE-A-2 545 295). Bereits geringfügige Schrägstellung oder Flachlegen des Ableitungssystems führt jedoch dazu, daß sofort verkeimte Flüssigkeitsreste an der Tropfkammerwand entlang direkt zu dem Tropfrohr gelangen und durch seine offene Spitze ein Keimtransport in die Körpersekretableitung und die Körperhöhle stattfindet. Die verkeimten Flüssigkeitsreste befinden sich in der Leitung zwischen Tropfkammerauslaß und Sammelbeutel, in die ein Klappenventil eingesetzt ist, das ein Zurückfließen von großen Urinmengen aus dem Sammelbeutel in die Tropfkammer verhindern soll, wenn das normalerweise hängende Ableitungssystem flachgelegt wird. Gegen ein Zurückfließen eingesickerter oder durch Anhaften zurückgebliebener kleiner Urinvolumina in die Tropfkammer vermag das Ventil aber natürlich nichts auszurichten, und die retrograde Keimwanderung nach oben in die Körpersekretableitung ist nur eine Zeitfrage.

Der Erfindung liegt die Aufgabe zugrunde, eine Tropfkammer in Sekretableitungs-, Sammel- und Meßsystemen, insbesondere für Urin, so auszubilden, daß eine Flüssigkeitsbenetzung der Spitze des Tropfrohres bei Schrägstellung oder Flachlegen des Systems durch von unten nach oben zurückfließende Flüssigkeitsreste wirkungsvoll vermieden wird, so daß die Spitze des Tropfrohres von Flüssigkeitsresten frei bleibt und die Tropfkammer ihre Funktion als Keimsperre zur Verhinderung aufsteigender Harnweginfektionen usw. zufriedenstellend erfüllt.

Diese Aufgabe wird bei einer Tropfkammer der eingangs genannten Art erfindungsgemäß dadurch gelöst, daß der Innenraum des Tropfkammergehäuses durch eine Wandung in einen an den senkrechten Teil der Wand des Gehäuses angrenzenden, oben geschlossenen und unten offenen Ringraum und in einen zweiten Raum, in den das Tropfrohr hineinragt, unterteilt ist.

Der Ringraum, der sich über den gesamten inneren Umfang des Gehäuses unterbrechungslos erstreckt, bildet eine oben geschlossene und unten offene Tasche, in die bei schräger oder waagerechter Tropfkammerhaltung die Restmenge des Körpersekrets hineinläuft, so daß sie im Wandbereich des Tropfkammergehäuses aufgefangen und an einem Vordringen zur Spitze des Tropfrohres gehindert wird. Es ergibt sich eine trockene Strecke zwischen der Spitze des Tropfers und dem Ventil, die den Zeitraum der Wanderung von Bakterien von unten nach oben gegenüber den bisherigen Tropfkammer-Konstruktionen um ein Mehrfaches verlängert. Kanalikulär erzeugte, das heißt retrograd aufgestiegene, Infektionen werden auf diese Weise bei einem geschlossenen Ableitungssystem für Körpersekret beträchtlich reduziert.

Um zusätzlich eine Benetzung der Spitze des Tropfrohres durch von oben nachtropfende frische Flüssigkeit zu verhindern und die Spitze völlig trocken und kontaminationsfrei zu halten, ist vorteilhaft die Körpersekretableitung oberhalb der Tropfkammer absperrbar ausgebildet. Zu diesem Zweck können eine Schieber- oder Quetschklemme oder ein Absperrhahn dienen. Auch kann die Ableitung durch Abknicken unterbrochen werden.

Vorteilhafterweise ist der Ringraum konzentrisch innerhalb des Gehäuses vorgesehen. Die Querschnittsform der Wandung des Ringraumes und der Wand des Gehäuses können einander entsprechen oder voneinander abweichen. Beispielsweise kann ein kreiszylinderförmiges Gehäuse mit einer im Querschnitt viereckigen Wandung des Ringraumes kombiniert werden oder umgekehrt, wenn dies aus fertigungstechnischen oder anderen Gründen erwünscht ist.

Die Innenflächen der Begrenzungen des Ringraumes sind zweckmäßigerweise glatt ausgebildet, damit die in den Ringraum eingeströmte Flüssigkeitsrestmenge bei Senkrechtstellung des Ableitungssystems möglichst vollständig in den unteren Auslaß der Tropfkammer strömt und nicht an der Wandung des Ringraumes haftet.

Das Tropfrohr kann koaxial oder seitlich versetzt zum Ringraum angeordnet sein. In beiden Fällen wird die Spitze des Tropfrohres gegen Flüssigkeitsbenetzung geschützt, weil die bei flachgelegtem Sammelsystem in der Tropfkammer nach oben fließende kleine Flüssigkeitsmenge durch den oberen Verschluß des Ringraumes in diesem zurückgehalten wird und nicht bis zur Spitze des Tropfrohres gelangen kann.

Die Wandung des Ringraumes ist vorteilhafterweise aus einer steifen Hülse gebildet, deren oberes Ende flüssigkeitsdicht mit der Wand des Gehäuses verbunden ist und die unterhalb der Spitze des Tropfrohres endet.

Die Hülse kann als gerader Zylinder beliebiger Querschnittsform oder kegelstumpfförmig oder ballig gewölbt oder hyperboloidförmig gestaltet sein.

Der obere Rand der Hülse kann mit dem oberen, waagerechten Teil der Wand des Gehäuses direkt verbunden sein. In diesem Falle umgibt die Hülse die Spitze des Tropfrohres und erstreckt sich vorteilhafterweise von ihrer oberen Befestigung über die Spitze des Tropfrohres hinaus ein Stück nach unten.

In weiterer vorteilhafter Ausgestaltung der Erfindung kann ein nach außen gerichteter, oberer Randflansch der Hülse mit dem senkrechten Teil der Wand des Gehäuses verbunden sein. In diesem Falle kann eine verhältnismäßig kurze Hülse verwendet werden, die im unteren Bereich der Tropfkammer jenseits der Spitze des Tropfrohres angeordnet werden kann. Durch den hierdurch erreichten axialen Abstand zwischen der Spitze des Tropfrohres und dem Ringraum wird eine weitere Verlangsamung der Bakterienwanderung nach oben erreicht. Außerdem ist der Materialverbrauch geringer.

Der Randflansch kann waagerecht oder trichterförmig verlaufen. Im letzteren Falle wirkt er für ein außermittig angeordnetes Tropfrohr als Prallfläche.

Der Mantel der Hülse kann knickfrei in seine untere Randzone übergehen, so daß die Räume auf beiden Seiten der Hülse an keiner Stelle verengt sind.

Die Tropfkammer kann mit einer Kammer verbunden sein, in der sich das Ventil befindet. Dies ist jedoch nicht zwingend, und das Ventil kann in der Tropfkammer oder in dem Sammelbeutel oder in einem Meßbehälter angeordnet sein. In diesem Falle entfällt eine gesonderte Ventilkammer, und die Tropfkammer ist unmittelbar an den Sammelbeutel oder einen Meßbehälter angeschlossen.

In der Zeichnung sind Ausführungsbeispiele der Erfindung schematisch dargestellt. Es zeigt

Fig. 1 eine Ansicht eines Ableitungssystems für Körpersekret mit einer Tropfkammer und einem Folienschlauchventil,

Fig. 2 einen Querschnitt durch das Tropfkammergehäuse längs der Linie II-II in Fig. 1,

Fig. 3 einen Längsschnitt durch ein Tropfkammergehäuse mit senkrechtem Membranventil,

Fig. 4 einen Längsschnitt durch ein Tropfkammergehäuse mit waagerechtem Membranventil,

Fig. 5 bis 7 Längsschnitte durch Tropfkammergehäuse mit außermittigem Tropfrohr und hängendem Folienschlauchventil, senkrechten Membranventil sowie waagerechtem Membranventil und mit einer abgewandelten Ringraumgestaltung,

Fig. 8 einen Längsschnitt eines Tropfkammergehäuses mit der Ringraumgestaltung nach den Fig. 5—7 jedoch mit zentralem Tropfrohr,

Fig. 9 einen Längsschnitt eines Tropfkammergehäuses mit kegelstumpfförmiger innerer Wandung des Ringraumes, und

Fig. 10 einen Längsschnitt eines Tropfkammergehäuses mit gewölbter, innerer Wandung des Ringraumes.

Ein Gehäuse 1 einer Tropfkammer mit kreiszylindrischem Querschnitt weist an dem oberen, waagerechten Teil 2 seiner Wand einen senkrechten Stutzen 3 auf, durch den ein Tropfrohr 4 mit einer Spitze 5 hindurchgeführt ist. Das Tropfrohr 4 steht mit einer Körpersekretableitung 6 in Verbindung. Der Bodenteil 7 des Gehäuses 1 ist trichterförmig abgeschrägt, und ein Stutzen 8 ist an eine Ventilkammer 9 angeschlossen, die — ebenso wie das Gehäuse 1 der Tropfkammer — vorzugsweise aus durchsichtigem Kunststoff hergestellt ist. Die Ventilkammer 9 weist auf der Vorderseite eine abgedeckte Filteröffnung 10 auf. In ihrem Inneren befindet sich ein Folienschlauchventil 11 aus einem flach zusammengelegten Folienschlauch, der durch Abschrägung seines unteren Randes eine Tropferspitze 12 erhalten hat. Die Tropferspitze befindet sich senkrecht über der Schräge einer Prallplatte 13, deren unteres Ende in einen Auslaßstutzen 14 der Ventilkammer 9 hineinragt. Der Auslaßstutzen 14 ist mittels eines aufgesteckten Verbindungsstükkes 15 mit einem Sammelbeutel 16 verbunden, der an nicht dargestellten Befestigungsmitteln einer von der Ventilkammer 9 ausgehenden Halterung 17 hängt.

Innerhalb des Gehäuses 1 der Tropfkammer ist ein kreiszylindrischer Ringraum 18 ausgebildet. Dieser ergibt sich durch eine kreiszylindrische Hülse 19, die aus steifem Material, insbesondere dem gleichen Material wie das Gehäuse 1 hergestellt ist, und deren oberer Rand an dem oberen Teil 2 des Gehäuses 1 befestigt ist. Die Hülse 19 umgibt das Tropfrohr 4 konzentrisch und erstreckt sich über die Spitze 5 des Tropfrohres hinaus nach unten. Sie endet mit Abstand über der trichterförmigen Schräge des Bodenteils 7 des Gehäuses 1, so daß das untere Ende des Ringraumes 18 offen ist.

Wenn das Ableitungssystem flachgelegt wird, fließt aus dem Folienschlauchventil 11 eine geringe Menge des Körpersekretes, insbesondere Urin, in das Gehäuse 1 zurück und läuft an der Wand des Gehäuses 1 entlang in den Ringraum 18. Da dieser oben geschlossen ist, wird ein Vordringen der Flüssigkeit zur Spitze 5 des Tropfrohres 4 verhindert, sie bleibt trocken und die Tropfkammer erfüllt ihren Zweck als Keimbarriere zuverlässig. Dies wird dadurch unterstützt, daß die Körpersekretableitung 6 oberhalb des Gehäuses 1 abgesperrt wird, damit keine frische Flüssigkeit nachtropfen kann und das Gehäuse 1 nicht aufgefüllt wird. Zur Unterbrechung der Ableitung 6 dienen an sich bekannte Schiebe- oder Quetschklemmen und dergleichen. Sobald das Ableitungssystem wieder in seine senkrecht hängende Gebrauchsstellung gebracht worden ist, rinnt der Flüssigkeitsrest aus dem Ringraum 18 nach unten und gelangt nach Öffnung der Ableitung 6 mit frischem Sekret aus dem Tropfrohr 4 durch das Folienschlauchventil 11 und die Ventilkammer 9 in den Sammelbeutel 16.

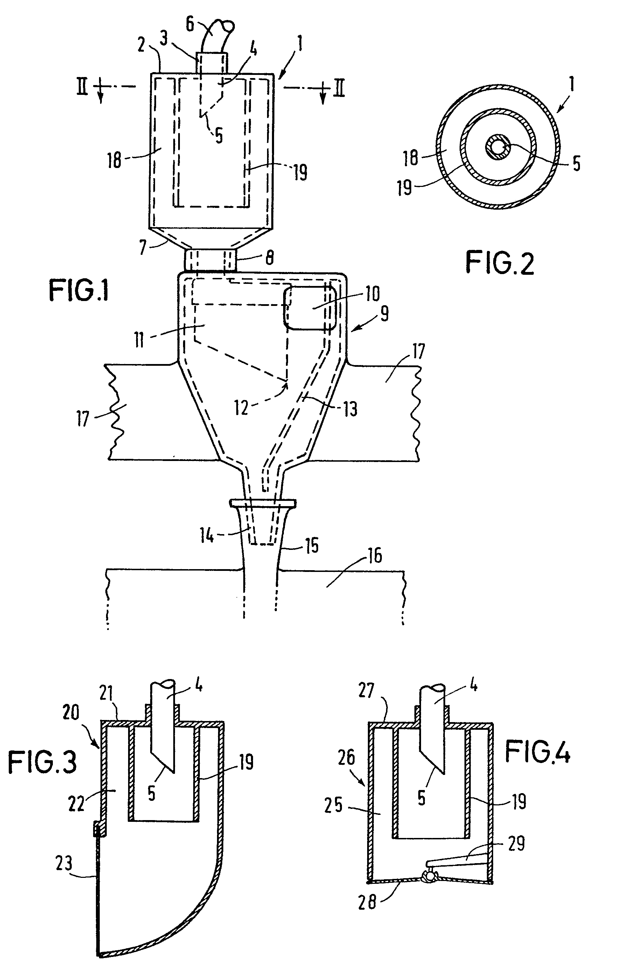

Bei dem Beispiel der Fig. 3 ist ein kreiszylindrisches Gehäuse 20 ebenfalls mit einer konzentrisch zur Tropfkammer und zum Tropfrohr 4 angeordneten kreiszylindrischen Hülse 19 versehen, die mit dem oberen Teil 21 der Wand des Gehäuses 20 verbunden ist, und die als innere Wandung eines unten offenen Ringraumes 22 dient. Das untere Ende der Hülse 19 endet oberhalb eines hängenden, senkrechten Membranventils 23, das sich an einer Seite der Tropfkammer 20 befindet, und das als Rückschlagventil ein Zurückströmen großer Mengen von Sekret aus dem Sammelbeutel 16 verhindert. Kleine Mengen von aufgrund einer verzögerten Ansprechzeit des Membranventils 23 in dem Gehäuse 20 zurückgehaltenen Körpersekrets rinnen bei flachgelegtem Ableitungssystem in den Ringraum 22 und gelangen auch bei dieser Ausführungsform nicht an die Spitze 5 des Tropfrohres 4.

Gemäß Fig. 4 ist ein kreiszylindrisches Tropfkammergehäuse 26 mit einer ebenfalls keiszylindrischen Hülse 19 ausgestattet, die gemeinsam mit der Wandung des Gehäuses 26 einen Ringraum 25 umschließt, der zu dem Tropfrohr 4 konzentrisch angeordnet ist. Die Hülse 19 ist am oberen Teil 27 der Wand des Gehäuses 26 befestigt. Ihr unteres Ende ragt über die Spitze 5 des Tropfrohres 4 hinaus und endet frei. Eine Bodenöffnung des Gehäuses 26 enthält ein waagerechtes Membranventil 28, das an einer Aufhängung 29 beweglich befestigt ist.

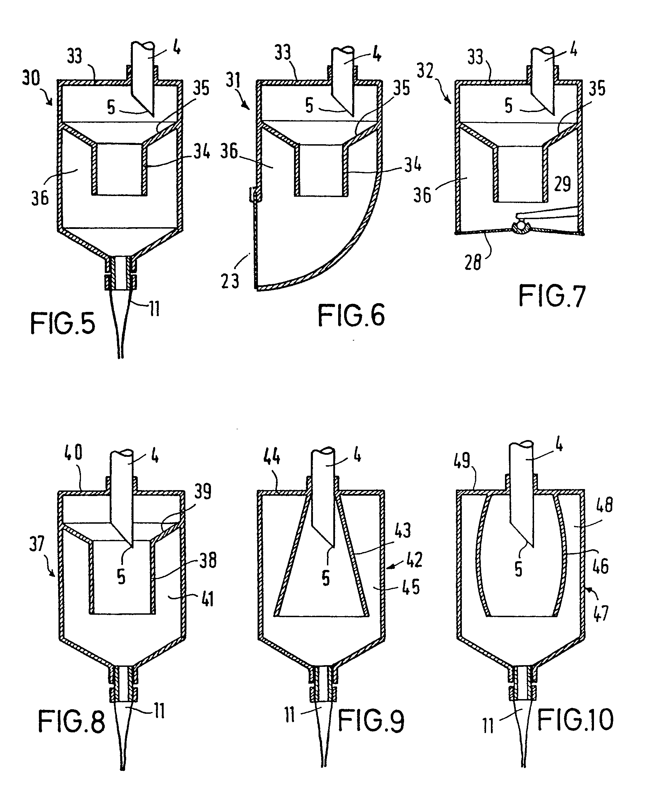

Bei den Beispielen der Fig. 5, 6 und 7 ist das Tropfrohr 4 außermittig in den oberen Teil 33 der Wand von Gehäusen 30, 31, 32 eingesetzt, deren untere Enden ein hängendes Folienschlauchventil 11 bzw. ein hängendes, senkrechtes Membranventil 23 bzw. ein waagerechtes Membranventil 28 aufweisen.

Eine im Verhältnis zu den Beispielen der Fig. 1 bis 4 kurze Hülse 34 ist unterhalb der Spitze 5 des Tropfrohres 4 angeordnet und mittels eines trichterförmig verlaufenden, nach außen gerichteten oberen Randflansches 35 mit dem senkrechten Teil der Wand der Gehäuse 30 bzw. 31 bzw. 32 verbunden. Der konzentrisch zu den Gehäusen 30 bzw. 31 bzw. 32 angeordnete Ringraum 36 verhindert den Rückfluß der geringen Flüssigkeitsmengen, die von den Ventilen 11, 23 und 28 durchgelassen werden. Die Spitze 5 des Tropfrohres 4 bleibt trocken, weil die Flüssigkeit in dem Ringraum 36 aufgehalten wird. Der schräge Randflansch 35 befindet sich senkrecht unter der Spitze 5 des Tropfrohres 4 und dient als Prallfläche, die ein Zurückspritzen des Körpersekretes gegen die Spitze 5 verhindert.

Fig. 8 zeigt eine Tropfkammer mit einem Gehäuse 37 und einer Hülse 38, die mittels eines oberen, trichterförmig schrägen Randflansches 39 mit dem senkrechten Teil der Wand des Gehäuses 37 verbunden ist. Der Randflansch 39 ist etwas oberhalb des Endes der Spitze 5 des Tropfrohres 4 vorgesehen, das sich in der Mitte des oberen, waagerechten Teiles 40 des Gehäuses 37 befindet. In diesem Falle dient der obere Randflansch 39 nicht als Prallfläche für die Spitze 5 des Tropfrohres 4. Die Funktion des zwischen der Wand des Gehäuses 37 und der Hülse 38 eingeschlossenen, unten offenen Ringraumes 41 entspricht jedoch derjenigen der Fig. 1 bis 7.

Ein anderes Beispiel einer Hülsenform ist in Fig. 9 gezeigt. In einem Gehäuse 42 ist ein Tropfrohr 4 mit einer Spitze 5 konzentrisch angeordnet. Das Tropfrohr 4 ist dicht von einer kegelstumpfförmigen Hülse 43 umgeben, deren verjüngtes Ende an dem oberen waagerechten Teil 44 des Gehäuses 42 befestigt ist. Ein nach unten offener Ringraum 45 hält von dem Ventil 11 durchgelassene Rastflüssigkeit von der Spitze 5 des Tropfrohres 4 zurück, wie im Zusammenhang mit den anderen Beispielen beschrieben wurde.

Eine ballig gewölbte Hülse 46 ist in Fig. 10 veranschaulicht. Sie umgibt ein konzentrisch zu ihr und der Wand des Gehäuses 47 angeordnetes Tropfrohr 4 und bildet die innere Wandung eines Ringraumes 48, der zurückströmende Restmengen von Flüssigkeit aufnimmt. Das obere Ende der gewölbten Hülse 46 ist an dem oberen, waagerechten Teil 49 des Gehäuses 47 befestigt. Ihr unteres Ende ist frei.

Die Beispiele der Fig. 3 bis 10 veranschaulichen schematisch Konstruktionen ohne Ventilkammer 9, bei denen das Ventil 11 bzw. 23 bzw. 28 in der Tropfkammer selbst oder in dem Sammelbeutel 16 oder in einem Meßbehälter angeordnet sein kann. In diesem Falle ist die Tropfkammer unmittelbar mit dem Sammelbeutel 16 oder einem Meßbehälter verbunden.

Die in den Figuren gezeigten Hülsenanordnungen und Ventile sind gegeneinander austauschbar und entsprechend ist die Länge der Hülsen veränderbar.

## Patentansprüche

1. Tropfkammer in einem Ableitungssystem für Körpersekret, insbesondere Urin, mit einem im wesentlichen starren Gehäuse (1; 20; 26; 30; 31; 32; 37; 42; 47), dessen unterer Auslaß (8) über ein Ventil (11; 23; 28) an einen Sammel- oder Meßbehälter (16) angeschlossen ist und dessen oberer Einlaß (3) ein in das Tropfkammergehäuse hineinragendes Tropfrohr (4) aufweist, das mit einer Körpersekretableitung (6) verbunden ist, dadurch gekennzeichnet, daß der Innenraum des Tropfkammergehäuses (1; 20; 26; 30; 31; 32; 37; 42; 47) durch eine Wandung (19; 34; 35; 38; 39; 43; 46) in einen an den senkrechten Teil der Wand des Gehäuses angrenzenden, oben geschlossenen und unten offenen Ringraum (18; 22; 25; 36; 41; 45; 48) und in einen zweiten Raum, in den das Tropfrohr (4), hineinragt, unterteilt ist.

2. Tropfkammer nach Anspruch 1, dadurch gekennzeichnet, daß der Ringraum (18; 22; 25; 36; 41; 45; 48) konzentrisch innerhalb des Gehäuses (1; 20; 26; 30; 31; 37; 42; 47) vorgesehen ist.

3. Tropfkammer nach Anspruch 1 oder 2, dadurch gekennzeichnet, daß die Querschnittsform der Wandung des Ringraumes (18; 22; 25; 36; 41; 45; 48) und der Wand des Gehäuses (1; 20; 26; 30; 31; 32; 37; 42; 47) einander entsprechen.

4. Tropfkammer nach Anspruch 1 oder 2, dadurch gekennzeichnet, daß die Querschnittsform der Wandung des Ringraumes und der Wand des Gehäuses voneinander abweichen.

5. Tropfkammer nach einem der Ansprüche 1 bis 4, dadurch gekennzeichnet, daß die Innenflächen der Begrenzungen des Ringraumes (18; 22; 25; 36; 41; 45; 48) glatt ausgebildet sind.

6. Tropfkammer nach einem der Ansprüche 1 bis 5, dadurch gekennzeichnet, daß das Tropfrohr (4) koaxial zum Ringraum (18; 22; 25; 41; 45; 48) angeordnet ist.

7. Tropfkammer nach einem der Ansprüche 1 bis 5, dadurch gekennzeichnet, daß das Tropfrohr (4) seitlich versetzt zum Ringraum (36) angeordnet ist.

8. Tropfkammer nach einem der Ansprüche 1 bis 7, dadurch gekennzeichnet, daß die Wandung des Ringraumes (18; 22; 25; 36; 41; 45; 48) aus einer steifen Hülse (19; 34; 38; 43; 46) gebildet ist, deren oberes Ende flüssigkeitsdicht mit der Wand des Gehäuses (1; 20; 26; 30; 31; 32; 37; 42; 47) verbunden ist und daß die Hülse (19; 34; 38; 43; 46) unterhalb der Spitze (5) des Tropfrohres (4) endet.

9. Tropfkammer nach Anspruch 8, dadurch gekennzeichnet, daß die Hülse (19; 34; 38) als gerader Zylinder gestaltet ist.

10. Tropfkammer nach Anspruch 8, dadurch gekennzeichnet, daß die Hülse (43) kegelstumpfförmig gestaltet ist.

11. Tropfkammer nach Anspruch 8, dadurch gekennzeichnet, daß die Hülse (46) ballig gewölbt gestaltet ist.

12. Tropfkammer nach Anspruch 8, dadurch gekennzeichnet, daß die Hülse hyperboloidförmig gestaltet ist.

13. Tropfkammer nach einem der Ansprüche 8 bis 12, dadurch gekennzeichnet, daß ein nach außen gerichteter oberer Randflansch (35; 39) der Hülse mit dem senkrechten Teil der Wand des Gehäuses (30; 31; 32; 37) verbunden ist.

14. Tropfkammer nach Anspruch 13, dadurch gekennzeichnet, daß der Randflansch waagerecht verläuft.

15. Tropfkammer nach Anspruch 13, dadurch gekennzeichnet, daß der Randflansch (35; 39) trichterförmig verläuft.

16. Tropfkammer nach einem der Ansprüche 13 bis 15, dadurch gekennzeichnet, daß der Randflansch (35; 39) im Bereich der Spitze (5) des Tropfrohres (4) oder ein Stück unter dieser angeordnet ist.

17. Tropfkammer nach einem der Ansprüche 8 bis 12, dadurch gekennzeichnet, daß der obere Rand der Hülse (19; 43; 48) mit dem oberen, waagerechten Teil (2; 21; 27; 44; 49) der Wand des Gehäuses (1; 20; 26; 42; 47) direkt verbunden ist.

18. Tropfkammer nach Anspruch 17, dadurch gekennzeichnet, daß die Hülse (19; 43; 46) sich von ihrer oberen Befestigung über die Spitze (5) des Tropfrohres (4) hinaus ein Stück nach unten erstreckt.

19. Tropfkammer nach den Ansprüchen 8 bis 18, dadurch gekennzeichnet, daß der Mantel der Hülse (19; 34; 38; 43; 46) knickfrei in seine untere Randzone übergeht.

20. Tropfkammer nach den Ansprüchen 1 bis 19, dadurch gekennzeichnet, daß die Körpersekretableitung (6) oberhalb des Gehäuses (1; 20; 26; 30; 31; 32; 37; 42; 47) absperrbar ausgebildet ist.

**Claims**

1. A dropping chamber in a drainage system for body secretion, more particularly urine, having a substantially rigid housing (1; 20; 26; 30; 31; 32; 37; 42; 47), whose lower outlet (8) is connected via a valve (11; 23; 28) to a collecting or measuring vessel (16) and whose upper inlet (3) has a dropping tube (4) projecting into the housing of the dropping chamber, which tube is connected to a body secretion drainage device (6), characterised in that the inside of the housing of the dropping chamber (1; 20; 26; 30; 31; 32; 37; 42; 47) is subdivided by a wall (19; 34; 35; 38; 39; 43; 46) into an annular chamber (18; 22; 25; 36; 41; 45; 48) adjacent to the vertical section of the wall of the housing, which chamber is sealed at the top and open at the bottom and into a second chamber into which the dropping tube (4) projects.

2. A dropping chamber according to claim 1, characterised in that the annular chamber (18; 22; 25; 36; 41; 45; 48) is arranged concentrically inside the housing (1; 20; 26; 30; 31; 32; 37; 42; 47).

3. A dropping chamber according to claim 1 or claim 2, characterised in that the cross-section shape of the wall of the annular chamber (18; 22; 25; 36; 41; 45; 48) and the wall of the housing (1; 20; 26; 30; 31; 32; 37; 42; 47) correspond to each other.

4. A dropping chamber according to claim 1 or claim 2, characterised in that the cross-section shape of the wall of the annular chamber and the wall of the housing are distinct from each other.

5. A dropping chamber according to one of claims 1 to 4, characterised in that the interior surfaces of the periphery of the annular chamber (18; 22; 25; 36; 41; 45; 48) are smooth.

6. A dropping chamber according to one of claims 1 to 5, characterised in that the dropping tube (4) is arranged co-axially to the annular chamber (18; 22; 25; 41; 45; 48).

7. A dropping chamber according to one of claims 1 to 5, characterised in that the dropping tube (4) is displaced laterally to the annular chamber (36).

8. A dropping chamber according to one of claims 1 to 7 characterised in that the wall of the annular chamber (18; 22; 25; 36; 41; 45; 48) is formed by a rigid sheath (19; 34; 38; 43; 46), whose upper end is connected in a liquid-tight manner to the wall of the housing (1; 20; 26; 30; 31; 32; 37; 42; 47) and that the sheath (19; 34; 38; 43; 46) ends below the tip (5) of the dropping tube (4).

9. A dropping chamber according to claim 8, characterised in that the sheath (19; 34; 38) is a straight cylinder.

10. A dropping chamber according to claim 8, characterised in that the sheath (43) is in the shape of a truncated cone.

11. A dropping chamber according to claim 8, characterised in that the sheath (46) is arched in a convex manner.

12. A dropping chamber according to claim 8, characterised in that the sheath is in the shape of a hyperboloid.

13. A dropping chamber according to one of claims 8 to 12, characterised in that an outwardly-directed upper peripheral flange (35; 39) of the sheath is connected to the vertical section of the wall of the housing (30; 31; 32; 37).

14. A dropping chamber according to claim 13, characterised in that the peripheral flange runs horizontally.

15. A dropping chamber according to claim 13, characterised in that the peripheral flange (35; 39) runs in a funnel-shaped manner.

16. A dropping chamber according to one of claims 13 to 15, characterised in that the peripheral flange (35; 39) is arranged in the area of the tip (5) of the dropping tube (4) or just below this.

17. A dropping chamber according to one of claims 8 to 12, characterised in that the upper periphery of the sheath (19; 43; 48) is directly connected to the upper horizontal section (2; 21; 27; 44; 49) of the wall of the housing (1; 20; 26; 42; 47).

18. A dropping chamber according to claim 17, characterised in that the sheath (19; 43; 46) extends from the upper fixing device thereof to just below the tip (5) of the dropping tube (4).

19. A dropping chamber according to claims 8 to 18 characterised in that the covering of the sheath (19; 34; 38; 43; 46) merges without buckling into the lower peripheral zone thereof.

20. A dropping chamber according to claims 1 to 19, characterised in that the body secretion drainage device (6) may be sealable above the housing (1; 20; 26; 30; 31; 32; 37; 42; 47).

**Revendication**

1. Dispositif goutte à goutte pour l'évacuation de fluides corporels, notamment l'urine, avec un corps essentiellement rigide (1; 20; 26; 30; 31; 32; 37; 42; 47) dont l'orifice de sortie inférieur (8) est relié, par l'intermédiaire d'un robinet (11; 23; 28), à un récipient de collecte ou de mesure (16) et dont l'orifice d'admission supérieur (3) comporte un tube à gouttes (4) qui s'engage dans le corps du dispositif goutte à goutte et est relié à un tuyau (6) d'évacuation de fluides corporels, caractérisé en ce que l'espace intérieur du corps du dispositif goutte à goutte (1; 20; 26; 30; 31; 32; 37; 42; 47) est subdivisé par une paroi (19; 34; 35; 38; 39; 43; 46) en un espace annulaire (18; 22; 25; 36; 41; 45; 48) fermé dans le haut et ouvert dans le bas, avoisinant la partie verticale de la paroi du corps et en un deuxième espace dans lequel pénètre le tube à gouttes (4).

2. Dispositif goutte à goutte selon la revendication 1, caractérisé en ce que l'espace annulaire (18; 22; 25; 36; 41; 45; 48) est prévu en position concentrique à l'intérieur du corps (1; 20; 26; 30; 31; 32; 37; 42; 47).

3. Dispositif goutte à goutte selon la revendication 1 ou 2, caractérisé en ce que la forme de la section transversale de la paroi de l'espace annulaire (18; 22; 25; 36; 41; 45; 48) et celle de la paroi du corps (1; 20; 26; 30; 31; 32; 37; 42; 47) se correspondent.

4. Dispositif goutte à goutte selon la revendication 1 ou 2, caractérisé en ce que la forme de la section transversale de la paroi de l'espace annulaire et celle de la paroi du corps diffèrent l'une de l'autre.

5. Dispositif goutte à goutte selon l'une des revendications 1 à 4, caractérisé en ce que les surfaces internes des délimitations de l'espace annulaire (18; 22; 25; 36; 41; 45; 48) sont lisses.

6. Dispositif goutte à goutte selon l'une des revendications 1 à 5, caractérisé en ce que le tube à gouttes (4) est disposé coaxialement par rapport à l'espace annulaire (18; 22; 25; 41; 48).

7. Dispositif goutte à goutte selon l'une des revendications 1 à 5, caractérisé en ce que le tube à gouttes (4) est décalé latéralement par rapport à l'espace annulaire (36).

8. Dispositif goutte à goutte selon l'une des revendications 1 à 7, caractérisé en ce que la paroi de l'espace annulaire (18; 22; 25; 36; 41; 45; 48) est constituée par un manchon rigide (19; 34; 38; 43; 46) dont l'extrémité supérieure est reliée, de manière étanche aux fluides, à la paroi du corps (1; 20; 26; 30; 31; 32; 37; 42; 47) et que le manchon (19; 34; 38; 43; 46) se termine au-dessous de la pointe (5) du tube à gouttes (4).

9. Dispositif goutte à goutte selon la revendication 8, caractérisé en ce que le manchon (19; 34; 38) se présente sous la forme d'un cylindre droit.

10. Dispositif goutte à goutte selon la revendication 8, caractérisé en ce que le manchon (43) se présente sous forme tronconique.

11. Dispositif selon la revendication 8, caractérisé en ce que le manchon (46) se présente sous forme bombée.

12. Dispositif selon la revendication 8, caractérisé en ce que le manchon se présente sous la forme d'un hyperboloïde.

13. Dispositif goutte à goutte selon les revendications 8 à 12, caractérisé en ce qu'un rebord supérieur du manchon (35; 39) dirigé vers l'extérieur se raccorde à la partie verticale de la paroi du corps (30; 31; 32; 37).

14. Dispositif goutte à goutte selon la revendication 13, caractérisé en ce que le rebord s'étend horizontalement.

15. Dispositif goutte à goutte selon la revendication 13, caractérisé en ce que le rebord (35; 39) a la forme d'un entonnoir.

16. Dispositif goutte à goutte selon les revendications 13 à 15, caractérisé en ce que le rebord (35; 39) est disposé dans la zone de la pointe (5) du tube à gouttes (4) ou un peu au-dessous de celle-ci.

17. Dispositif goutte à goutte selon les revendications 8 à 12, caractérisé en ce que le bord supérieur du manchon (19; 43; 48) se raccorde directement à la partie horizontale supérieure (2; 21; 27; 44; 49) de la paroi du corps (1; 20; 26; 42; 47).

18. Dispositif goutte à goutte selon la revendication 17, caractérisé en ce que, à partir de sa fixation supérieure, le manchon (19; 43; 46) s'étend un peu vers le bas au-delà de la pointe (5) du tube à gouttes (4).

19. Dispositif goutte à goutte selon les revendications 8 à 18, caractérisé en ce que l'enveloppe du manchon (19; 34; 38; 43; 46) se raccorde sans coude à sa zone périphérique inférieure.

20. Dispositif goutte à goutte selon les revendications 1 à 19, caractérisé en ce que le tuyau (6) d'évacuation des fluides corporels, au-dessus du corps (1; 20; 26; 30; 31; 32; 37; 42; 47) est agencé de manière à pouvoir être isolé.

FIG.1

FIG.2

FIG.3

FIG.4

FIG.5

FIG.6

FIG.7

FIG.8

FIG.9

FIG.10